# EUROPÄISCHE PATENTSCHRIFT

(11) **EP 1 059 905 B1**
(45) Veröffentlichungstag und Bekanntmachung des Hinweises auf die Patenterteilung: **23.07.2003**
(21) Anmeldenummer: 99903529.8
(22) Anmeldetag: 12.02.1999
(51) Int. Cl.: A61F 9/02

(54) **SICHTSCHEIBE FÜR EINE SKIBRILLE**
LOOKING GLASS FOR SKI GOGGLES
VERRE POUR LUNETTES DE SKI

(30) Priorität: 05.03.1998 AT 37998
(43) Veröffentlichungstag der Anmeldung: 20.12.2000
(73) Patentinhaber: SILHOUETTE INTERNATIONAL SCHMIED GMBH & CO. KG., 4021 Linz (AT)
(72) Erfinder: SPINDELBALKER, Rupert, A-4040 Puchenau (AT)
(74) Vertreter: Hübscher, Gerhard, Dipl.-Ing.
(86) Internationale Anmeldenummer: AT9900036
(87) Internationale Veröffentlichungsnummer: WO99044555

(56) Entgegenhaltungen:
- WO-A-92/07630
- DE-A- 2 317 088
- DE-B- 1 091 871
- GB-A- 524 196
- GB-A- 2 284 679
- US-A- 1 562 350
- US-A- 5 018 223
- US-A- 5 642 530

## Beschreibung

### Technisches Gebiet

Die Erfindung bezieht sich auf eine doppelwandige Sichtscheibe für eine Skibrille mit einer gekrümmten Außenscheibe und einer mit Abstand von der Außenscheibe angeordneten, ebenfalls gekrümmten Innenscheibe aus einer Cellulosepropionatfolie, die mit der Außenscheibe über ein randseitiges Dichtungsband verbunden ist.

### Stand der Technik

Um bei Skibrillen das Beschlagen der Sichtscheibe mit kondensierendem Wasserdampf möglichst zu vermeiden, ist es bekannt, doppelwandige Sichtscheiben einzusetzen, die in einem gegenseitigen Abstand miteinander über ein randseitiges Dichtungsband verbunden sind. Die durch die Luftschicht zwischen der Außen- und der Innenscheibe bedingte Wärmedämmung ermöglicht eine entsprechende Temperaturdifferenz zwischen den beiden Scheiben, was wegen der damit verbundenen Taupunktverlagerung im Vergleich mit einer einwandigen Sichtscheibe zu einer deutlich geringeren Neigung zum Beschlagen der Sichtscheibe führt, insbesondere wenn die beiden Scheiben aus einem Werkstoff mit einer Oberflächenbeschaffenheit gefertigt sind, die das Beschlagen mit kondensierendem Wasserdampf erschwert, wie dies beim Einsatz von Cellulosepropionatfolien der Fall ist. Nachteilig bei den doppelwandigen Sichtscheiben aus Cellulosepropionatfolien ist allerdings, daß diese Folien eine vergleichsweise geringe mechanische Festigkeit aufweisen und daher nicht als kratzfest angesehen werden können. Außerdem kommt es trotz der doppelwandigen Ausbildung der Sichtscheibe und dem vorteilhaften Beschlagsverhalten bei ungünstigen Temperatur- und Feuchtigkeitsverhältnissen zu einem die Sicht durch die Skibrille beeinträchtigenden Beschlagen der Sichtscheibe mit kondensierendem Wasserdampf.

Eine doppelwandige Sichtscheibe gemäß dem Oberbegriff des Anspruchs 1 ist aus der US-A-1 562 350 bekannt.

### Darstellung der Erfindung

Der Erfindung liegt somit die Aufgabe zugrunde, eine doppelwandige Sichtscheibe für eine Skibrille der eingangs geschilderten Art so auszubilden, daß nicht nur die mechanische Festigkeit der Sichtscheibe gesteigert, sondern auch das Beschlagsverhalten verbessert werden kann.

Die Erfindung löst die gestellte Aufgabe dadurch, daß sowohl die aus Polycarbonat gespritzte Außenscheibe als auch die durch ein Vakuumtiefziehen der Cellulosepropionatfolie gefertigte Innenscheibe kugelförmig gekrümmt sind und daß die Außenscheibe auf ihrer Innenseite eine hygroskopische Beschichtung aufweist.

Durch die kugelförmige Krümmung sowohl der Außen- als auch der Innenscheibe wird im Vergleich zu einer herkömmlichen Sichtscheibe, die mit einer zylindrischen Krümmung zur Anpassung der Skibrille an die Kopfform versehen ist, das von der Skibrille zwischen dem Kopf des Trägers und der Sichtscheibe umschlossene Luftvolumen vergrößert, was sich vorteilhaft auf das Beschlagsverhalten der Skibrille auswirkt, und zwar in einem überraschenden Ausmaß. Da die Außenscheibe aus Polycarbonat in einem Spritzgußverfahren gefertigt wird, kann die angestrebte kugelförmige Krümmung über eine entsprechende Ausgestaltung der Spritzgußform vergleichsweise einfach erzielt werden. Schwierigkeiten ergeben sich jedoch bezüglich der kugelförmigen Krümmung der Innenscheibe, die ja durch eine aus optischen Gründen nicht ohne weiteres umformbare Cellulosepropionatfolie gebildet wird. Erst durch ein Vakuumtiefziehen der eingesetzten Cellulosepropionatfolie kann die angestrebte kugelförmige Krümmung der Innenscheibe ohne Einbußen hinsichtlich der optischen Qualität der Sichtscheibe sichergestellt werden, wenn auf eine entsprechend geringe Molekülverstreckung bei dem Tiefziehvorgang geachtet wird.

Um eine hohe Bruchsicherheit mit einer guten Kratzfestigkeit verbinden zu können, ist es bekannt, Sichtscheiben aus Polycarbonat zu fertigen. Mit dem Einsatz von Sichtscheiben aus Polycarbonat muß allerdings auf das besonders gute Beschlagsverhalten von Cellulosepropionatfolien verzichtet werden. Da nach der erfindungsgemäßen Ausbildung der Sichtscheibe eine Außenscheibe aus Polycarbonat mit einer Innenscheibe aus einer Cellulosepropionatfolie kombiniert und die Außenscheibe auf ihrer Innenseite zusätzlich mit einer hygroskopischen Beschichtung versehen wird, kann sich dieser sonst bei Sichtscheiben aus Polycarbonat auftretende Nachteil nicht auswirken, so daß sich durch das Zusammenwirken der angegebenen Maßnahmen in Verbindung mit der kugelförmigen Krümmung der Sichtscheibe auch bei ungünstigen Temperatur- und Feuchtigkeitsverhältnissen eine geringe Neigung zum Beschlagen einer solchen Sichtscheibe ergibt.

### Kurze Beschreibung der Zeichnungen

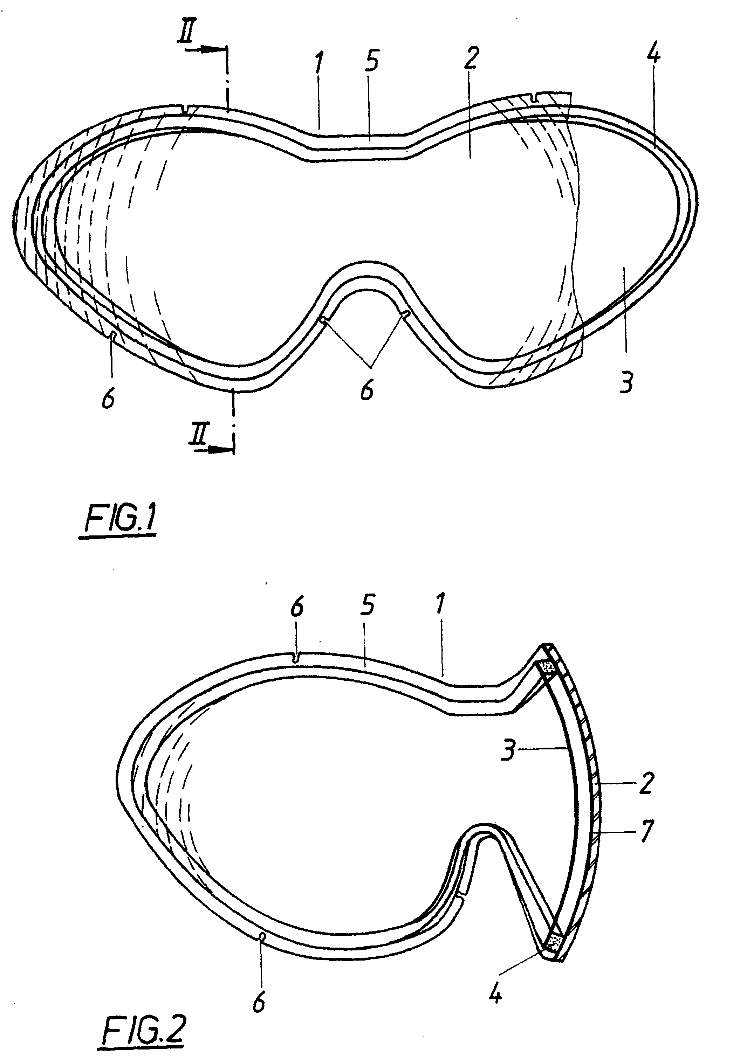

In der Zeichnung ist der Erfindungsgegenstand beispielsweise dargestellt. Es zeigen
- Fig. 1: ein erfindungsgemäße Sichtscheibe für eine Skibrille in einer zum Teil aufgerissenen schematischen Draufsicht und
- Fig. 2: einen Schnitt nach der Linie II-II der Fig. 1.

### Bester Weg zur Ausführung der Erfindung

Die dargestellte Sichtscheibe 1 besteht aus einer Außenscheibe 2 aus Polycarbonat und einer Innenscheibe 3 aus einer Cellulosepropionatfolie, die über ein randseitiges, umfangsgeschlossenes Dichtungsband 4 mit der Außenscheibe 2 verbunden ist. Dieses Dichtungsband 4 ist aus einem geschlossenporigen Schaumstoff gefertigt und mit den beiden Scheiben 2 und 3 verklebt. Die Außenscheibe 2 ragt mit einem Randsteg 5 über das Dichtungsband 4 vor und bildet im Bereich des Randsteges 5 Aussparungen 6, mit deren Hilfe die Sichtscheibe 1 in einer rahmenartigen Halterung der Skibrille in herkömmlicher Weise verankert wird.

Zum Unterschied zu herkömmlichen doppelwandigen Sichtscheiben ist die erfindungsgemäße Sichtscheibe 1 zur Anpassung der Skibrille an die Kopfform nicht zylindrisch, sondern kugelförmige gekrümmt, so daß sowohl die Außenscheibe 2 als auch die Innenscheibe 3 zumindest angenähert Kugelflächen bilden. Durch diese kugelförmige Krümmung der Sichtscheibe 1 wird das von der Skibrille umschlossene Luftvolumen zwischen der Sichtscheibe 1 und dem Brillenträger vergrößert, was das Beschlagsverhalten der Skibrille im Zusammenwirken mit den übrigen Maßnahmen in einem überraschend hohen Ausmaß verbessert. Diese zusätzlichen Maßnahmen bezüglich der Verbesserung des Beschlagsverhaltens sind darin zu sehen, daß das gegenüber der Innenscheibe aus Cellulosepropionat ungünstigere Beschlagsverhalten der Außenscheibe 2 aus Polycarbonat durch eine hygroskopische Beschichtung 7 auf der Innenseite der Außenscheibe 2 ausgeglichen wird. In diesem Zusammenhang ist zu berücksichtigen, daß die Luftschicht zwischen den beiden Scheiben 2 und 3 nicht feuchtigkeitsdicht abgeschlossen werden kann, weil die eingesetzten Kunststoffe keine vollständige Dampfsperre bilden.

Während die Außenscheibe 2 aus Polycarbonat in einem herkömmlichen Spritzgußverfahren hergestellt werden kann, liegt die Innenscheibe 3 in Form einer Cellulosepropionatfolie vor, die hinsichtlich der kugelförmigen Krümmung Schwierigkeiten bereitet, wenn keine optischen Qualitütseinbußen in Kauf genommen werden sollen. Aus diesem Grunde wird die Innenscheibe 3 durch ein Vakuumtiefziehverfahren kugelförmig gewölbt, wobei unter Bedachtnahme auf eine geringe Molekülverstreckung optische Verzerrungen vermieden werden können.

## Patentansprüche

1. Doppelwandige Sichtscheibe (1) für eine Skibrille mit einer gekrümmten Außenscheibe (2), wobei die Außenscheibe (2) auf ihrer Innenseite eine hygroskopische Beschichtung (7) aufweist, und einer mit Abstand von der Außenscheibe (2) angeordneten, ebenfalls gekrümmten Innenscheibe (3) aus einer Cellulosepropionatfolie, die mit der Außenscheibe (2) über ein randseitiges Dichtungsband (4) verbunden ist, **dadurch gekennzeichnet, daß** sowohl die aus Polycarbonat gespritzte Außenscheibe (2) als auch die durch ein Vakuumtiefziehen einer Cellulosepropionatfolie gefertigte Innenscheibe (3) kugelförmig gekrümmt sind.

## Claims

1. A double-walled eye-protecting lens (1) for ski goggles comprising a curved outer lens (2), the outer lens (2) having a hygroscopic coating (7) on the inside, and a likewise curved inner lens (3) disposed at a distance from the outer lens (2) and made from a cellulose propionate sheet connected to the outer lens (2) by a sealing strip (4) round the edge, **characterised in that** the outer lens (2), injection-moulded from polycarbonate, and the inner lens (3), made by deep-drawing a cellulose propionate sheet in vacuo, are both spherically curved.

## Revendications

1. Verre (1) à double paroi pour lunettes de ski, avec un verre extérieur (2) incurvé, où le verre extérieur (2) présente, sur sa face intérieure, un revêtement (7) hygroscopique, et avec un verre intérieur (3) également incurvé, également disposé à distance du verre extérieur (2) et réalisé en feuille de propionate de cellulose, relié au verre extérieur (2) par l'intermédiaire d'une bande d'étanchéité (4) située en bordure, **caractérisé en ce que**, tant le verre extérieur (2) moulé par injection en poly carbonate, qu'également le verre intérieur (3) fabriqué par étirage profond sous vide d'une feuille de propionate de cellulose, sont incurvés en forme de sphère.
